(19) Europäisches Patentamt
European Patent Office
Office européen des brevets

(11) Veröffentlichungsnummer: **0 193 806**
**A2**

(12) # EUROPÄISCHE PATENTANMELDUNG

(21) Anmeldenummer: **86102253.1**

(22) Anmeldetag: **21.02.86**

(51) Int. Cl.⁴: **C 07 D 251/16**

(30) Priorität: **05.03.85 DE 3507751**

(43) Veröffentlichungstag der Anmeldung: **10.09.86 Patentblatt 86/37**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Priesnitz, Uwe, Dr., Severinstrasse 58, D-5650 Solingen (DE)**
Erfinder: **Riebel, Hans-Jochem, Dr., In der Beek 92, D-5600 Wuppertal 1 (DE)**

(54) **Verfahren zur Herstellung von 2-Cyanamino-1,3,5-triazinen.**

(57) 2-Cyanamino-1,3,5-triazine der allgemeinen Formel (I), welche als Zwischenprodukte zur Herstellung von herbizid wirksamen Guanidin-Derivaten verwendet werden können,

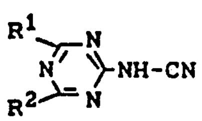

(I)

in welcher
$R^1$ für Alkyl steht und
$R^2$ für Alkoxy, Alkylamino oder Dialkylamino steht,
können mit guten Ausbeuten hergestellt werden, indem man Oxime der Formel (II)

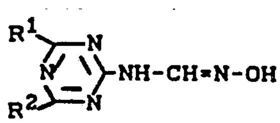

(II)

mit Methansulfonsäurechlorid in Gegenwart von Säureakzeptoren und in Gegenwart von Verdünnungsmitteln umsetzt.

Ein Verfahren zur Herstellung der neuen Zwischenprodukte (II), ausgehend von bekannten 2-Amino-1,3,5-triazinen über ebenfalls neue 2-Formamidino-Derivate, wird ebenfalls beschrieben.

EP 0 193 806 A2

BAYER AKTIENGESELLSCHAFT 5090 Leverkusen, Bayerwerk

Konzernverwaltung RP
Patentabteilung Bi/mö
IVa/ZP

# Verfahren zur Herstellung von 2-Cyanamino-1,3,5-triazinen

Die vorliegende Erfindung betrifft ein neues Verfahren zur Herstellung von 2-Cyanamino-1,3,5-triazinen, sowie neue Zwischenprodukte hierfür. Die Verfahrensprodukte sind zum Teil bekannt und können als Zwischenprodukte zur Herstellung von Herbiziden und Pflanzenwachstumsregulatoren verwendet werden.

Es ist bereits bekannt, daß 2-Cyanamino-1,3,5-triazine durch Umsetzung von Alkalimetall- oder Erdalkalimetall-Salzen von Cyanamid mit den entsprechenden 2-Halogen-1,3,5-triazinen erhalten werden (vergl. z. B. DE-OS 3 334 455, EP-A-121 082).
Dieses Verfahren ist jedoch wegen unbefriedigender Herstellungsmethoden für die 2-Halogen-1,3,5-triazine nur begrenzt anwendbar.

Es wurde nun gefunden, daß man 2-Cyanamino-1,3,5-triazine der allgemeinen Formel (I)

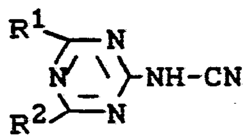

(I)

in welcher

$R^1$ für Alkyl steht und

$R^2$ für Alkoxy, Alkylamino oder Dialkylamino steht,

erhält, wenn man Oxime der Formel (II)

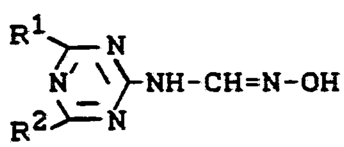

(II)

in welcher

$R^1$ und $R^2$ die oben angegebenen Bedeutungen haben,

mit Methansulfonsäurechlorid in Gegenwart von Säureakzeptoren und in Gegenwart von Verdünnungsmitteln umsetzt.

Überraschenderweise gelingt es mit Hilfe des erfindungsgemäßen Verfahrens, eine Dehydratisierung der Oxime durchzuführen. Nach dem Stand der Technik ist eine Dehydratisierung chemisch ähnlicher Verbindungen mit z. B. Phosphoroxychlorid nicht oder nur in sehr schlechten Ausbeuten möglich (J.Het. Chem. 19, 577 (1982)).

Bevorzugt werden mit Hilfe des erfindungsgemäßen Verfahrens die Verbindungen der Formel (I) hergestellt, in welcher

$R^1$ für Alkyl mit 1 bis 6 Kohlenstoffatomen steht und

$R^2$ für Alkoxy mit 1 bis 6 Kohlenstoffatomen, Alkylamino oder Dialkylamino mit jeweils 1 bis 6 Kohlenstoffatomen im Alkylteil steht.

Besonders bevorzugt werden diejenigen Verbindungen der Formel (I) hergestellt, in welcher

$R^1$ für $C_1$-$C_4$-Alkyl steht und

$R^2$ für $C_1$-$C_4$-Alkoxy, $C_1$-$C_4$-Alkylamino oder Di-($C_1$-$C_4$)-Alkylamino steht.

Ganz besonders bevorzugt werden diejenigen Verbindungen der Formel (I) hergestellt, in welcher

$R^1$ für Methyl, Ethyl, n-Propyl, i-Propyl oder n-Butyl steht und

$R^2$ für Methoxy, Ethoxy, n-Propoxy, i-Propoxy, n-Butoxy, (Di)Methylamino, (Di)Ethylamino, (Di)n-Propylamino, (Di)i-Propylamino, (Di)n-Butylamino, (Di)i-Butylamino, (Di)sec.-Butylamino, tert.-Butylamino, Ethylmethylamino, Methyl-n-propylamino, Methyl-i-propylamino, Methyl-n-butylamino, Methyl-i-butylamino, Ethyl-n-propylamino, Ethyl-i-propylamino, Ethyl-n-butylamino, Ethyl-i-butylamino, n-Propyl-i-propylamino, n-Propyl-n-butylamino, n-Propyl-i-butylamino, i-Propyl-n-butylamino, i-Propyl-i-butylamino und n-Butyl-i-butylamino steht.

Verwendet man beispielsweise für das erfindungsgemäße Verfahren N-(4-Ethoxy-6-methyl-1,3,5-triazin-2-yl)-formamidoxim und Methansulfonsäurechlorid als Ausgangsprodukte, so kann die Reaktion durch das folgende Formelschema wiedergegeben werden:

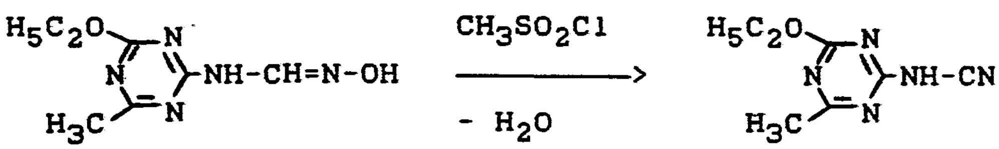

Die als Ausgangsstoffe für das erfindungsgemäße Verfahren zu verwendenden Oxime sind durch die Formel (II) allgemein definiert. In dieser Formel stehen vorzugsweise

$R^1$ für Alkyl mit 1 bis 6 Kohlenstoffatomen und

$R^2$ für Alkoxy mit 1 bis 6 Kohlenstoffatomen, Alkylamino oder Dialkylamino mit jeweils 1 bis 6 Kohlenstoffatomen im Alkylteil.

Besonders bevorzugt sind diejenigen Verbindungen der Formel (II), in welcher

$R^1$ für $C_1$-$C_4$-Alkyl steht und

$R^2$ für $C_1$-$C_4$-Alkoxy, $C_1$-$C_4$-Alkylamino oder Di-($C_1$-$C_4$)-Alkylamino steht.

Ganz besonders bevorzugt sind diejenigen Verbindungen der Formel (II), in welcher

$R^1$ für Methyl, Ethyl, n-Propyl, i-Propyl oder n-Butyl steht und

$R^2$ für Methoxy, Ethoxy, n-Propoxy, i-Propoxy, n-Butoxy, (Di)Methylamino, (Di)Ethylamino, (Di)n-Propylamino, (Di)i-Propylamino, (Di)n-Butylamino, (Di)i-Butylamino, (Di)sec.-Butylamino und tert.-Butylamino steht.

Die Verbindungen der Formel (II) sind neu. Sie lassen sich nach bekannten Methoden, z. B. folgendermaßen herstellen, indem man Formamidine der Formel (III)

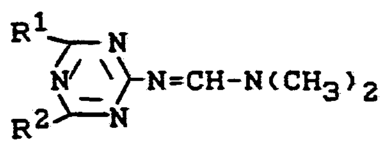

(III)

in welcher

$R^1$ und $R^2$ die oben angegebenen Bedeutungen haben,

mit Hydroxylamin-Hydrochlorid in Gegenwart eines Verdünnungsmittels, wie z. B. Methanol oder Dimethylformamid und bei Temperaturen zwischen 15 °C und 30 °C umsetzt.

Die Formamidine der Formel (III) sind ebenfalls neu. Sie lassen sich nach bekannten Methoden herstellen, z. B. durch Umsetzung von 2-Amino-1,3,5-triazinen der Formel (IV)

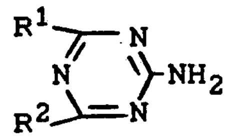

(IV)

in welcher

$R^1$ und $R^2$ die oben angegebenen Bedeutungen haben,

mit Dimethylformamid-dimethylacetal und in Gegenwart eines Verdünnungsmittels wie z. B. Toluol, bei Temperaturen zwischen 80 °C und 120 °C.

Die Verbindungen der Formel (IV) sind bekannte Verbindungen der organischen Chemie.

Die Formamidine, die als Ausgangsprodukte für die Herstellung der Verbindungen der Formel (II) eingesetzt werden, sind durch die Formel (III) allgemein definiert. In dieser Formel stehen $R^1$ und $R^2$ vorzugsweise bzw. insbesondere bevorzugt für diejenigen Reste, welche bevorzugt bzw. insbesondere bevorzugt für diese Substituenten in Formel (II) angegeben sind.

Das erfindungsgemäße Verfahren zur Herstellung der Verbindungen der Formel (I) wird in Gegenwart von inerten Verdünnungsmitteln durchgeführt.

Hierzu gehören insbesondere aliphatische Kohlenwasserstoffe wie Hexan, ferner Ether, wie Diethyl- und Dibutylether, Glykoldimethylether und Diglykoldimethylether, Tetrahydrofuran und Dioxan, Amide, wie z. B. Dimethylformamid, Dimethylacetamid und N-Methylpyrrolidon, sowie Dimethylsulfoxid, Tetramethylensulfon und Hexamethylphosphorsäuretriamid.

Das erfindungsgemäße Verfahren wird in Gegenwart von Säureakzeptoren durchgeführt. Als Säureakzeptoren können alle üblichen Säurebindemittel Verwendung finden. Besonders bewährt haben sich Alkalicarbonate, wie Natrium- und Kaliumcarbonat, ferner aliphatische, aromatische oder heterocyclische Amine, beispielsweise Triethylamin, Trimethylamin, Dimethylanilin, Dimethylbenzylamin und Pyridin.

Das erfindungsgemäße Verfahren wird im allgemeinen bei Temperaturen zwischen 0 °C und +160 °C durchgeführt. Bevorzugt wird der Bereich zwischen 20 °C und 140 °C. Die Umsetzungen werden im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird    in einem geeigneten Verdünnungsmittel in Gegenwart eines Säureakzeptors durchgeführt und

das Reaktionsgemisch wird bei der erforderlichen Temperatur gerührt. Danach gibt man ein organisches Lösungsmittel, z. B. Methylenchlorid, zu und arbeitet die organische Phase wie üblich durch Waschen, Trocknen und Abdestillieren des Lösungsmittels auf.

Die nach dem erfindungsgemäßen Verfahren herzustellenden 2-Cyanamino-1,3,5-triazine können als Zwischenprodukte für die Herstellung von Guanidin-Derivaten, die als Herbizide und Pflanzenwuchsregulatoren wirksam sind, eingesetzt werden (vergl. EP-OS 121 082).

Herstellungsbeispiele

Beispiel 1

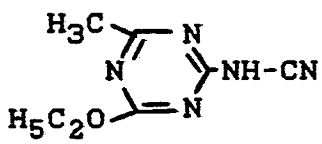

Eine Mischung aus 20 g (0,1 Mol) N-(4-Ethoxy-6-methyl-1,3,5-triazin-2-yl)-formamidoxim in 400 ml Glykoldimethylether wird mit 12,5 g (0,11 Mol) Methansulfonsäurechlorid versetzt und 2 Stunden unter Rückfluß erhitzt. Nach Abkühlen auf 20 °C wird mit einer Mischung aus 8 g Triethylamin und 20 ml Glykoldimethylether versetzt und 1 Stunde unter Rückfluß erhitzt. Anschließend wird mit Salzsäure neutralisiert, bis zur Trockne eingedampft und in 100 ml Methylenchlorid aufgenommen.

Die Konzentration an 2-Cyanamino-4-ethoxy-6-methyl-1,3,5,-triazin wird mittels "äußerer Standardmethode" analytisch bestimmt. Bezogen auf diese Konzentration beträgt die Ausbeute 50 % der Theorie

Die Zielverbindung ist nach chromatographischer Reinigung hochrein herstellbar und ist durch ihr $^1$H-NMR-Spektrum charakterisiert.

$^1$H-NMR ($CDCl_3$) $\delta$ = 1.38 (t, $CH_3$-$CH_2$); 2.36 (S, $CH_3$); 4.38 (q, $CH_2$-$CH_3$); $H_{1/2}$ = 5Hz; 6.17 (br, - NH) ppm.

Fp: 195°C (Zers.).

Analog Beispiel 1 können die folgenden Verbindungen der Formel (I) erhalten werden:

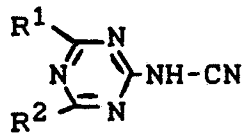

(I)

Beispiel 2

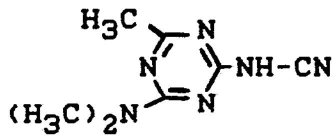

0193806

Ausgangsverbindungen der Formel (II)

Beispiel (II-1)

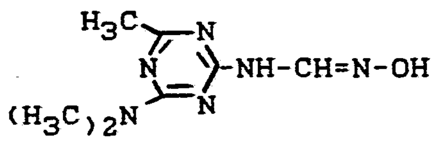

Zu einer Lösung von 52 g (0,25 Mol) N,N-Dimethyl-N'-(4-dimethylamino-6-methyl-1,3,5-triazin-2-yl)-formamidin in 400 ml Methanol werden 21 g (0,3 Mol) Hydroxylamin-Hydrochlorid gegeben und 3 Stunden bei 20 °C nachgerührt.

Nach dem Abfiltrieren erhält man 49 g (100 % der Theorie) N-(4-Dimethylamino-6-methyl-1,3,5-triazin-2-yl)-formamidoxim vom Schmelzpunkt 256 °C.

Analog Beispiel (II-1) können die folgenden Verbindungen der Formel (II) hergestellt werden:

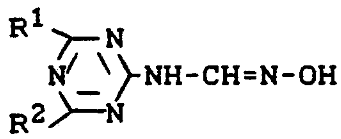

(II)

Beispiel (II-2)

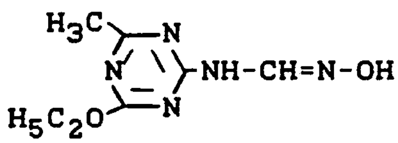

Fp: 233 °C

Beispiel (II-3)

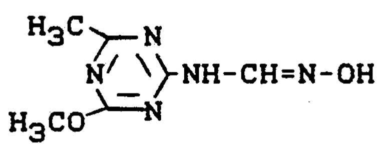

Fp > 220 °C

Ausgangsverbindungen der Formel (III)

Beispiel (III-1)

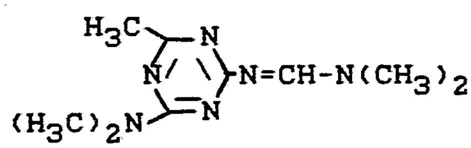

Eine Mischung aus 50 g (0,327 Mol) 2-Amino-4-dimethylamino-6-methyl-1,3,5-triazin, 55 ml (0,59 Mol) Dimethylformamid-dimethylacetal und 300 ml Toluol wird 5 Stunden unter Rückfluß erhitzt und anschließend eingeengt.

Man erhält 67 g (100 % der Theorie) N,N-Dimethyl-N'-(4-dimethylamino-6-methyl-1,3,5-triazin-2-yl)-formamidin vom Schmelzpunkt 61 °C.

0193806

Analog Beispiel (III-1) können die folgenden Verbindungen der Formel (III) erhalten werden:

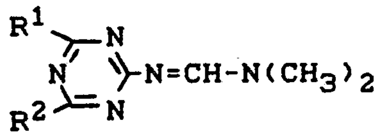

(III)

Beispiel (III-2)

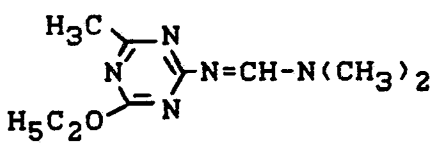

Fp: 48 °C

Beispiel (III-3)

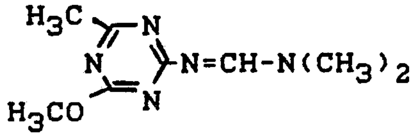

Fp: 58 °C

Patentansprüche

1) Verfahren zur Herstellung von 2-Cyanamino-1,3,5-triazinen der allgemeinen Formel (I)

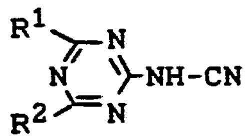

(I)

in welcher

$R^1$ für Alkyl steht und

$R^2$ für Alkoxy, Alkylamino oder Dialkylamino steht,

dadurch gekennzeichnet, daß man Oxime der Formel (II)

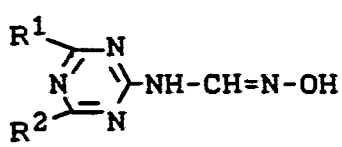

(II)

in welcher

$R^1$ und $R^2$ die oben angegebenen Bedeutungen haben,

mit Methansulfonsäurechlorid, in Gegenwart von Säureakzeptoren und in Gegenwart von Verdünnungsmitteln umsetzt.

2) Oxime der Formel (II)

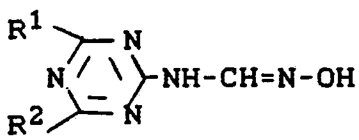

(II)

in welcher

$R^1$ für Alkyl steht und

$R^2$ für Alkoxy, Alkylamino oder Dialkylamino steht.

3) Verfahren zur Herstellung von Oximen der Formel (II), gemäß Anspruch 2, dadurch gekennzeichnet, daß man Formamidine der Formel (III)

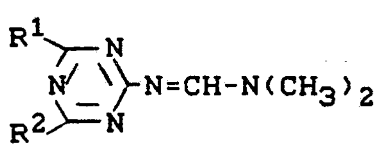

(III)

in welcher

$R^1$ und $R^2$ die in Anspruch 2 angegebenen Bedeutungen haben,

mit Hydroxylamin-Hydrochlorid in Gegenwart eines Verdünnungsmittels und bei Temperaturen zwischen 15 °C und 30 °C umsetzt.

4) Formamidine der Formel (III)

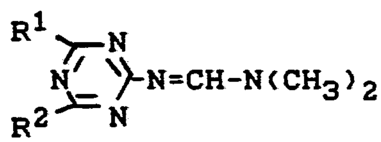 (III)

in welcher

$R^1$ für Alkyl steht und

$R^2$ für Alkoxy, Alkylamino oder Dialkylamino steht.

5) Verfahren zur Herstellung von Formamidinen der Formel (III), gemäß Anspruch 4, dadurch gekennzeichnet, daß man 2-Amino-1,3,5-triazine der Formel (IV)

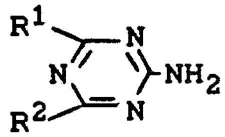

(IV)

in welcher

$R^1$ und $R^2$ die in Anspruch 4 angegebene Bedeutung haben,

mit Dimethylformamid-dimethylacetal und in Gegenwart eines Verdünnungsmittels bei Temperaturen zwischen 80 °C und 120 °C umsetzt.